# EUROPÄISCHE PATENTSCHRIFT

(11) **EP 0 248 362 B1**
(45) Veröffentlichungstag und Bekanntmachung des Hinweises auf die Patenterteilung: **28.07.1993**
(21) Anmeldenummer: 87107804.4
(22) Anmeldetag: 29.05.1987
(51) Int. Cl.: C07K 3/08

(54) **Verfahren zur Gewinnung aktiver Proteine aus einer biologisch inaktiven Form**
Process for the production of an active protein from a biologically inactive form
Procédé de production d'une protéine active à partir d'une forme biologiquement inactive

(30) Priorität: 04.06.1986 DE 3618817
(43) Veröffentlichungstag der Anmeldung: 09.12.1987
(73) Patentinhaber: BEHRINGWERKE Aktiengesellschaft, 35001 Marburg (DE)
(72) Erfinder: Hermann, Reinhard, Dr., NL-2716 LZ Zoetermeer (NL)

(56) Entgegenhaltungen:
- EP-A- 0 150 066
- JOURNAL OF CHROMATOGRAPHY, Band 109, 1975, Seiten 399-402, Elsevier Scientific Publsihing Co., Amsterdam, NL; I.P. GRIFFITH: "The use of Sephadex LH-20 to separate dodecyl sulphate and buffer salts from denatured proteins"
- METHODS IN ENZYMOLOGY, Band 91, 1983, Seiten 278-281, Academic press, Inc.; R.I. CHRISTOPHERSON: "Desalting protein solutions in a centrifuge column"
- ANGEWANDTE CHEMIE, Band 96, Nr. 6, 1984, Seiten 385-402, Verlag Chemie GmbH, Weinheim, DE; R. JAENICKE: "Proteinfaltung und Proteinassoziation"

## Beschreibung

Die Erfindung betrifft ein Verfahren zur Überführung eines Proteins aus einer Konformation, in der es biologisch inaktiv ist, in eine biologisch aktive Form. Im Falle eines denaturierten natürlichen Proteins könnte dieser Vorgang auch als Renaturierung bezeichnet werden.

### Hintergrund der Erfindung und Stand der Technik

Reinigungs- sowie Sterilisierungsverfahren an Proteinpräparaten können zur teilweisen Denaturierung des eingesetzten Proteins führen. Bisher war es vor allem aus wirtschaftlichen Erwägungen günstiger, denaturiertes Protein abzutrennen und zu verwerfen. Gentechnisch in Procaryonten hergestelltes Protein liegt weitgehend in biologisch inaktiver Form vor.

Um die Ausbeute an "nativem" Protein, das heißt solchem mit korrekter räumlicher Struktur und der biologischen Aktivität des natürlichen Proteins zu erhöhen, müssen die Polypeptidkette zunächst zum "random coil" aufgefaltet und eventuell vorliegende falsche Disulfidbrücken reduziert werden. Dies geschieht üblicherweise durch Inkubation in mindestens 4 mol/l Guanidinhydrochloridlösung oder mindestens 6 mol/l Harnstofflösung, gegebenenfalls mit Zusatz eines Reduktionsmittels wie Dithiothreitol (DTT). Anschließend wurde die Ausbildung der korrekten Proteinstruktur bislang durch Verdünnen (mindestens 1:40) oder Dialyse gegen eine "physiologische" Pufferlösung bewirkt.

Beide Methoden sind großtechnisch kaum anwendbar. Die Verdünnung ohnehin großer Volumina mit anschließender Rekonzentrierung ist zeitaufwendig, umständlich und teuer. Ähniches gilt auch für die Dialyse großer Volumina. Überdies senkt langsames Entfernen von denaturierendem Agens die Reaktivierungs-Ausbeute erheblich, weil Nebenreaktionen wie Aggregationen vorzugsweise im Bereich intermediärer Konzentrationen an denaturierendem Agens ablaufen.

### Beschreibung und Gegenstand der Erfindung

Es wurde überraschenderweise gefunden, daß die Nachteile der Verfahren des Standes der Technik dadurch vermieden werden können, daß aus der das denaturierende Agenz und das Protein enthaltenden Lösung das denaturierende Agenz entfernt wird, indem man die Lösung durch ein Material mit Molekularsieb-Eigenschaften passieren läßt, welches ein Medium enthält, in welchem das Protein seine biologisch aktive Form annimmt, wobei die Porengröße dieses Molekularsieb-Materials so gewählt wird, daß das denaturierende Agenz, jedoch nicht das Protein eindringen kann.

Gegenstand der Erfindung ist deshalb ein Verfahren zur Herstellung einer biologisch aktiven räumlichen Form eines Proteins aus einer biologisch inaktiven räumlichen Form, dadurch gekennzeichnet, daß das Protein unter Zusatz eines Denaturierungsmittels gelöst und dadurch in die "random coil"-Form übergeführt wird und die Lösung durch ein Material mit Molekularsiebeigenschaften passieren gelassen wird, das ein flüssiges Medium enthält, in welchem das Protein eine biologisch aktive räumliche Form annehmenkann, und wobei dieses Material mit Molekularsiebeigenschaften so gewählt wird, daß die Moleküle des Denaturierungsmittels, jedoch nicht die Proteinmoleküle eindringen können, und wobei das Passieren der Lösung durch das Material mit Molekularsiebeigenschaften durch eine über die Schwerkraft hinausgehende Kraft bewirkt wird.

Das Molekularsieb kann sich beispielsweise in einer Säule oder einem Zentrifugenkorb befinden.

Vorzugsweise wird, nachdem das Molekularsieb mit dem Medium äquilibriert wurde, in welchem das Protein eine biologisch aktive Form annimmt, der Teil des Mediums, welcher sich nicht in den Poren des Molekularsiebs befindet (das "Außenvolumen"), abgetrennt.
Dies wird vorteilhaft durch Zentrifugation erreicht, kann aber beispielsweise auch durch Ausblasen mittels eines Gases oder Absaugen durch Anlegen eines Vakuums herbeigeführt werden.

Dann wird die Lösung, die das "entfaltete" Protein und das Denaturierungsmittel enthält, auf das Molekularsieb aufgetragen. Das Durchdringen der Lösung durch das Molekularsiebmaterial sollte durch eine über die Schwerkraft hinausgehende Kraft bewirkt werden. Vorzugsweise geschieht dies durch Zentrifugation, aber auch durch Gasdruck oder Vakuum.
Falls eine Zentrifugation angewandt wird, entspricht der Arbeitsablauf weitgehend der bekannten Technik einer Korb- oder Siebzentrifugation.

Ein solches Molekularsieb kann eines der für die Gelfiltration bekannten Materialien sein, das gegen das Denaturierungsmittel chemisch resistent ist, beispielsweise ^{R}Sephadex G-25, DG 6P (^{R}Bio Rad, USA) oder "controlled pore glass". Die Porengröße wird so gewählt, daß das Denaturierungsmittel, nicht aber das Protein in die Matrix eindringen kann. Die Ausschlußgröße wird meist bei einem Mr von 6 000 bis 10 000 liegen (Mr=Molekulargewicht).

Es wird mit einer Lösung, in der das Protein seine aktive Form annimmt, vorzugsweise einem Puffer, äquilibriert und zum Beispiel in eine vorzugsweise zentrifugierbare Säu1e oder einen Zentrifugierkorb übergeführt. Die nicht in den Poren der Matrix (nicht "im Innenvolumen") befindliche Lösung wird vorzugsweise durch Zentrifugation bei etwa 300-1000xg abgetrennt. Anschließend wird die Denaturierungsmittel enthaltende Proteinlösung (Volumen kleiner als 30 % des Gelvolumens) aufgetragen. Während die Moleküle der Denaturierungslösung gegen den Puffer im Innenvolumen ausgetauscht werden kann, bleiben Proteine (Molekulargewicht größer als 6000) im Außenvolumen. Durch erneute Zentrifugation (2 min, 300-1000xg) können die Proteine quantitativ in ein Auffanggefäß abgeschleudert werden. Dies kann entsprechend bekannter Entsalzungsverfahren mittels Zentrifugation in einer Korbzentrifuge geschehen. Denaturierungsmittel sind dann nicht mehr nachweisbar. Das Volumen der erhaltenen Lösung entspricht dann dem Volumen der aufgetragenen Lösung.

Die Abtrennung des Äquilibrierungsmediums im Außenvolumen als auch die Beschleunigung des Austauschs des Äquilibrierungsmediums im Innenvolumen gegen die Denaturierungsmittel enthaltende Proteinlösung kann auch mittels Gasdruck oder Vakuum bewirkt werden.

Das erfindungsgemäße Verfahren ermöglicht, ein Protein auch aus großen Volumina eines Denaturierungsmediums schnell, quantitativ und ohne Verdünnung in ein Medium, in welchem das Protein eine aktive Form annimmt, zu überführen und hohe Ausbeuten an aktivem Protein zu erzielen.

Auf die beschriebene Weise kann kommerziell sonst nicht verwertbares Proteinmaterial verwendbar gemacht werden.

Das Verfahren zeichnet sich durch Einfachheit, Schnelligkeit und Reproduzierbarkeit aus. Zur Durchführung kann auf vorhandene und konventionelle Apparaturen und Materialien zurückgegriffen werden. Das Gelmaterial kann nach Gebrauch regeneriert und beispielsweise Guanidinhydrochlorid rückgewonnen werden. Die Proteinkonzentration bleibt unverändert.

Denaturierte Proteine sind speziell Proteine in nichtnativem Zustand nach einer Hitzebehandlung, zum Beispiel zur Inaktivierung von infektiösem Material, nach einer Säurebehandlung, zum Beispiel einer Säurespaltung von gentechnisch erhaltenen Fusionsproteinen, nach einer Behandlung mit strukturzerstörenden Agentien, zum Beispiel im Verlauf von Reinigungs-, Extraktions- oder Solubilisierungsschritten sowie bei der Inaktivierung infektiösen Materials oder nach gentechnischer Herstellung und dabei auftretender falscher Konformation und/oder falscher Ausbildung von Disulfidbrücken.

Geeignete Denaturierungsmittel zur völligen Auffaltung des Proteins sind zum Beispiel hochmolare Lösungen von Guanidiniumsalzen, Harnstoff oder anderen chaotropen Molekülen, gegebenenfalls in Gegenwart eines Reduktionsmittels, beispielsweise 50-150 mmol/l Dithiothreitol (DTT). Die Konzentrationen betragen üblicherweise und beispielsweise für Guanidinsalze 4-7, für Harnstoff 6-8 und für Isothiocyanat 6-8 mol/l und für 2-Chlor-ethanol etwa 400 ml/l.

Die Ausbildung der biologisch aktiven (nativen) Struktur wird herbeigeführt durch schnelle Überführung in einen Puffer, der die native Struktur begünstigt.

Die Schnelligkeit der Überführung ist wichtig für eine hohe Ausbeute. Im beschriebenen Verfahren liegt die Zeit im Sekunden- bis Minutenbereich.

Geeignete Aktivierungspuffer sind beispielsweise Phosphat- oder Trispuffer oder als "Good-buffers" (Biochem. (1966) 15, 467-477) bekannte Puffer, die auf den pH-Wert maximaler Aktivität oder Stabilität des Proteins eingestellt sind.

Die denaturierenden Moleküle werden schnell und quantitativ vorzugsweise durch Zentrifugation abgetrennt.

Das Medium, in dem das Protein seine biologisch aktive Konformation annimmt, ist meist ein Puffer und weist eine für die Stabilität des Proteins vorteilhafte Zusammensetzung auf (enthält zum Beispiel Phosphate, Sulfate, Citrate). Es kann als weitere Zusätze enthalten zum Beispiel Zucker, Peptide oder Proteine zur Stabilisierung der nativen Struktur oder Detergentien, zum Beispiel ^{R}Tween 20 oder NP40 zur Verhinderung der Adhäsion, der Aggregation und/oder zur Solvatisierung, und/oder SH-Reagentien oder Redox-Systeme zum Beispiel DTT oder Glutathion/Glutathiondisulfid (GSH/GSSG) zur Einstellung des für die Ausbildung korrekter Disulfidbrücken optimalen Redoxpotentials.

Reproduzierbare Redoxbedingungen werden durch Entgasen und Stickstoff-Sättigen der Puffer gewährleistet.

Korbzentrifugation im Sinne der Erfindung ist jede Zentrifugationstechnik in jedem Volumenbereich mit jeglicher apparativer Ausrüstung, bei der ein beliebiges Makromolekül (in Puffer A) mittels Zentrifugation durch ein mit Puffer B äquilibriertes, gegebenenfalls vorzentrifugiertes Gelfiltrationsmedium in Puffer B überführt wird.

Die folgenden Beispiele erläutern die Erfindung.

### Beispiel 1

Denaturierung von aktivem "mouse-GM-colony stimulating factor" (Mu GM-CSF, rekombinant aus Hefe) in Guanidin, und Reaktivierung.

Jeweils 3 Proben von je 1 µg einer von 5 Lösungen von GM-CSF 5 verschiedener Glycosylierungsgrade (A bis E) wurden in 40 µl 6 mol/l Guanidin-HCl in phosphatgepufferter Kochsalzlösung (PBS), pH 7,2 aufgenommen und die Lösung 60 Minuten bei Raumtemperatur gehalten.

^{R}Sephadex G-25 wurde in 15 Röhrohen mit einem Volumen von 0,5 ml eingefüllt und je 5 mit entgastem, stickstoffgesättigtem PBS ohne Zusatz oder enthaltend 1 mmol/l DTT oder 0,02 ml/100 ml ^{R} Tween 20 äquilibriert. Die im Außenvolumen befindliche Flüssigkeit wurde bei 700xg (5 Minuten) abgeschleudert. Auf je eines dieser jeweils 5 mit PBS, mit PBS + DTT oder Tween äquilibrierten Röhrohen wurde jeweils eine der 5 Lösungen von GM-CSF (A bis E) aufgebracht.

Durch zweiminütige Zentrifugation bei 700 g unmittelbar nach Auftrag der 15 verschiedenen Lösungen auf die 15 Röhrohen erhielt man 15 mal jeweils 40 µl einer guanidinfreien GM-CSF Präparation.

Alle 15 Proben wurden über Nacht bei Raumtemperatur unter Stickstoff aufbewahrt und dann die Aktivität im Knochenmarkstest oder auf einer GM-CSF-abhängigen Zellinie bestimmt.

Die Aktivität der guanidin-behandelten Proben erreichte je nach Reaktivierungspuffer bis zu 100 % der Ausgangsaktivität (etwa 2x10⁷ Einheiten (U)/mg). Die Ausbeute an Protein, bestimmt in der SDS-Elektophorese und im Western-Blot war praktisch quantitativ.

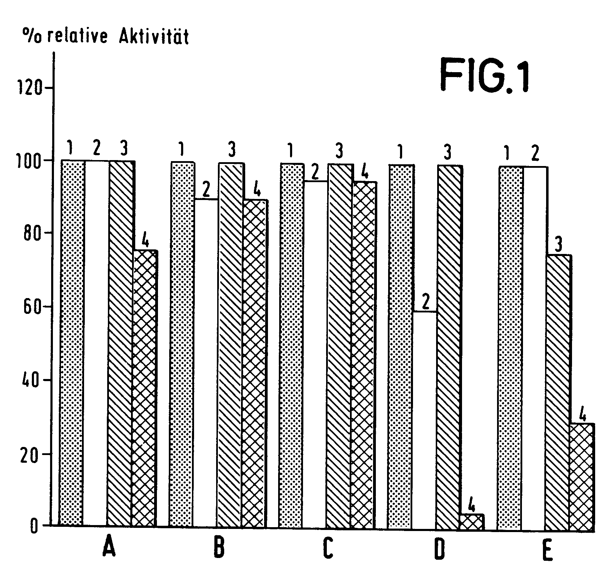

Die Ergebnisse sind in Figur 1 dargestellt. In diesem Diagramm bezeichnet 1 jeweils die Säule für die Aktivität der Lösung des nicht mit Denaturierungsmittel behandelten und reaktivierten GM-CSF und 2 die für den in PBS, 3 die für den in PBS und ^{R}Tween und 4 die für den in PBS und DTT reaktivierten GM-CSF. A bis E bezeichnen jeweils eine Gruppe der Aktivitäten für eine von fünf GM-CSF-Präperationen mit verschiedenem Ausmaß der Glycosylierung.

### Beispiel 2

Entfaltung in 6 mol/l Guanidin und Aktivierung von aggregiertem rekombinantem Human GM-CSF.

2 Präparationen von durch Säurespaltung eines Fusionsproteins aus E . coli erhaltenem aggregiertem, lyophilisiertem Human-GM-CSF (A und B) wurden jeweils in 6 mol/l Guanidin-HCl in PBS gelöst und 60 Minuten bei Raumtemperatur inkubiert. Der CSF-Anteil am Gesamtprotein war etwa 20 microgramm/100 microgramm.

Die weitere Behandlung wurde wie in Beispiel ausgeführt.

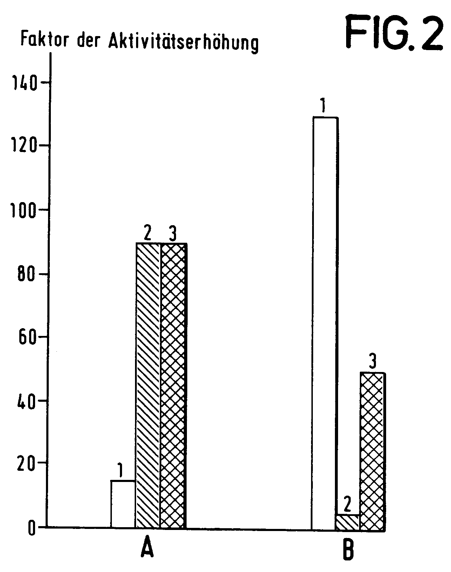

Die Ergebnisse sind in Fig. 2 dargestellt.

Die guanidin-behandelten Proben erreichten eine bis zu 130fache Aktivität des Ausgangs wertes . Es wurde eine maximale spezifische Aktivität von 2 x 10⁷ Einheiten/mg bestimmt. Als Reaktivierungspuffer wurden PBS (Säulen Nr.1), PBS + 0,02 % ^{R}Tween 20 (Säulen Nr.2) oder PBS + 0,1 mmol/l Dithiothreitol (Säulen Nr.3) verwendet.

### Beispiel 3

Entfaltung in 8 mol/l Harnstoff und Aktivierung von aggregiertem, inaktivem rekombinantem Human-GM-CSF

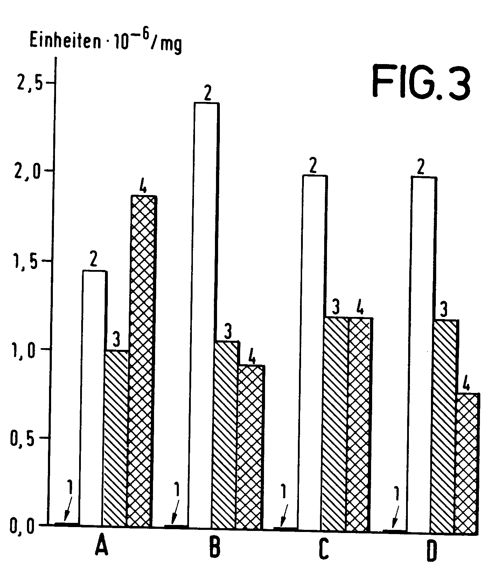

Aggregiertes, lyophilisiertes Human-GM-CSF aus E. coli (3 Proben nach Säurespaltung bezeichnet als A, B und C sowie eine Probe ungespalten bezeichnet als D; CSF-Gehalt etwa 20 µg/100 µg Gesamtprotein) wurde in 8 mol/l Harnstoff in Tris HCl, pH 8,0 gelöst (Proteinkonzentration 1 mg/ml, Probenvolumen je 0,5 ml) und 60 min. bei Raumtemperatur inkubiert. Die weitere Behandlung wurde wie in Beispiel 1 ausgeführt. Als Reaktivierungspuffer wurde PBS (Säulen Nr.1) oder PBS + 0,02 % Tween (Säulen Nr.2), PBS + "low"* GSH (Säulen Nr.4) oder PBS + "high"*GSH (Säulen Nr. 3) nach Säurespaltung verwendet. In allen Fällen wurde aus gänzlich inaktivem Material spezifische Aktivität nahe oder gleich der maximalen spezifischen Aktivität erhalten (Fig. 3).
* "low GSH": 25 µm GSH/50 µm GSSG (entspricht extrazellulärem Redoxpotential)
"high GSH": 5 mM GSH/0,1 mM GSSG (entspricht intrazellulärem Redoxpotential)

Auch ungespaltenes Fusionsprotein zeigt erhebliche biologische Aktivität (D). Die spezifische Aktivität nach der Reaktivierung wurde zu 1-2x10⁷ Einheiten/mg bestimmt.

### Beispiel 4

Entfaltung und komplette Reduktion aller Disulfidbrücken von aggregiertem, inaktivem, rekombinantem Human GM-CSF, Rückfaltung und Reoxidation zum biologisch aktiven Material wie in Beispiel 3.

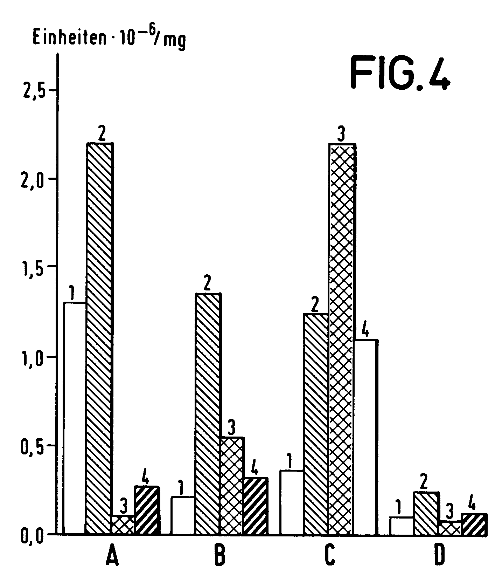

Denaturierung und Reduktion in 8 mol/l Harnstoff in Tris HCl, pH 8,0 + 0,15 mol/l Dithiothreitol; Faltung zum biologisch aktiven Produkt in PBS (Säulen Nr.1) oder PBS + 0,1 % Human-Serum Albumin (Säulen Nr.2), PBS + high GSH (Säulen Nr.3) oder PBS + low GSH (Säulen Nr.4) (Fig. 4).

## Patentansprüche

1. Verfahren zur Herstellung einer biologisch aktiven räumlichen Form eines Proteins aus einer biologisch inaktiven räumlichen Form, dadurch gekennzeichnet, daß das Protein unter Zusatz eines Denaturierungsmittels gelöst und dadurch in die "random coil"-Form übergeführt wird und die Lösung durch ein Material mit Molekularsiebeigenschaften passieren gelassen wird, das ein flüssiges Medium enthält, in welchem das Protein eine biologisch aktive räumliche Form annehmen kann und wobei dieses Material mit Molekularsiebeigenschaften so gewählt wird, daß die Moleküle des Denaturierungsmittels, jedoch nicht die Proteinmoleküle eindringen können, und wobei das Passieren der Lösung durch das Material mit Molekularsiebeigenschaften durch eine über die Schwerkraft hinausgehende Kraft bewirkt wird.

2. Verfahren nach Anspruch 1, dadurch gekennzeichnet, daß der nicht im Innenvolumen des Materials mit Molekularsiebeigenschaften befindliche Teil des flüssigen Mediums, in welchem das Protein eine biologisch aktive räumliche Form annehmen kann, durch Zentrifugieren, Ausblasen oder Absaugen entfernt wird, bevor die Proteinlösung mit diesem Material in Kontakt gebracht wird.

3. Verfahren nach Anspruch 1, dadurch gekennzeichnet, daß das Passieren der Flüssigkeit durch Zentrifugieren beschleunigt wird.

4. Verfahren nach Anspruch 1, dadurch gekennzeichnet, daß das Material mit Molekularsiebeigenschaften ein für die Gelfiltration bekanntes Material ist, das gegen das Denaturierungsmittel chemisch resistent ist.

5. Verfahren nach Anspruch 1, dadurch gekennzeichnet, daß das Material mit Molekularsiebeigenschaften ^{R}Sephadex G-25, ^{R}BioRad DG 6P oder "controlled pore glass" ist.

6. Verfahren nach Anspruch 1, dadurch gekennzeichnet, daß das flüssige Medium, in welchem das Protein eine biologisch aktive Form annimmt, ein wässriger Puffer ist, der für die Aktivität und Stabilität des Proteins günstige Agenzien, SH-Reagenzien oder Netzmittel enthält.

## Claims

1. A process for the preparation of a spatial form, which has biological activity, of a protein from a biologically inactive spatial form, which comprises the protein being dissolved with the addition of a denaturing agent and thus converted into the random coil form, and the solution being allowed to pass through a material which has molecular sieve properties and contains a liquid medium in which the protein can assume a spatial form which has biological activity, and this material which has molecular sieve properties being selected so that the molecules of the denaturing agent, but not the protein molecules, can penetrate in, and the passage of the solution through the material which has molecular sieve properties being brought about by a force which exceeds the force of gravity.

2. The process as claimed in claim 1, wherein the portion of the liquid medium which is not located in the internal volume of the material having molecular sieve properties and in which the protein can assume a biologically active spatial form is removed by centrifugation, blowing out or sucking out before the protein solution is contacted with this material.

3. The process as claimed in claim 1, wherein the rate of passage of the liquid is increased by centrifugation.

4. The process as claimed in claim 1, wherein the material having molecular sieve properties is a material which is known for gel filtration and is chemically resistant to the denaturing agent.

5. The process as claimed in claim 1, wherein the material having molecular sieve properties is ^{R}Sephadex G-25, ^{R}BioRad DG 6P or controlled pore glass.

6. The process as claimed in claim 1, wherein the liquid medium in which the protein assumes a biologically active form is an aqueous buffer which contains agents favorable to the activity and stability of the protein, SH reagents or wetting agents.

## Revendications

1. Procédé pour la production d'une forme spatiale biologiquement active d'une protéine, à partir d'une forme spatiale biologiquement inactive, caractérisé en ce que l'on dissout la protéine avec addition d'un agent dénaturant, et on la met ainsi sous la forme d'enroulement au hasard, puis on fait passer la solution à travers un matériau à propriétés de tamis moléculaire, qui contient un milieu liquide dans lequel la protéine peut adopter une forme spatiale biologiquement active, et ce matériau à propriétés de tamis moléculaire étant choisi de manière que les molécules de l'agent dénaturant puissent pénétrer, mais non les molécules de protéine, et le passage de la solution à travers le matériau à propriétés de tamis moléculaire étant provoqué par une force excédant la force de la pesanteur.

2. Procédé selon la revendication 1, caractérisé en ce que la partie du milieu liquide ne se trouvant pas dans le volume interne du matériau à propriétés de tamis moléculaire, milieu dans lequel la protéine peut adopter une forme spatiale biologiquement active, est éliminée par centrifugation, soufflage ou aspiration, avant la mise en contact de la solution de protéine avec ce matériau.

3. Procédé selon la revendication 1, caractérisé en ce que le passage du liquide est accéléré par centrifugation.

4. Procédé selon la revendication 1, caractérisé en ce que le matériau à propriétés de tamis moléculaire est un matériau connu pour la filtration sur gel, qui est chimiquement résistant à l'agent dénaturant.

5. Procédé selon la revendication 1, caractérisé en ce que le matériau à propriétés de tamis moléculaire est du Sephadex® G-25, du DG-6P de Bio Rad® ou du verre à taille de pores déterminée ("controlled pore glass").

6. Procédé selon la revendication 1, caractérisé en ce que le milieu liquide dans lequel la protéine adopte une forme biologiquement active est un tampon aqueux qui contient des agents mouillants, des réactifs SH ou des agents favorables à l'activité et à la stabilité de la protéine.
